# EUROPEAN PATENT APPLICATION

(11) **EP 1 652 521 A1**
(43) Date of publication of application: **03.05.2006**
(21) Application number: 04024929.4
(22) Date of filing: 20.10.2004
(51) Int. Cl.: A61K 31/40, A61K 31/4184, A61K 31/17, A61K 31/404, A61K 31/519, A61P 15/10

(54) **Pharmaceutical composition of sildenafil and a large conductance Ca(2+) activated K+ channels activator**

(71) Applicant: LABORATORIOS DEL DR. ESTEVE, S.A., 08041 Barcelona (ES)
(72) Inventor: The designation of the inventor has not yet been filed

(57) **Abstract**

The present invention refers to a pharmaceutical composition of sildenafil and a large conductance Ca(2+) activated K+ channels activator and its use for the treatment of sexual dysfunction in man and woman, sexual disorders in man, erectile dysfunction or impotence.

## Description

### Field of the invention

The present invention refers to a pharmaceutical composition of sildenafil and a large conductance Ca(2+) activated K+ channels activator and its use for the treatment of sexual dysfunction in man and woman, sexual disorders in man, erectile dysfunction or impotence.

### Background of the invention

The treatment of sexual dysfunction especially impotence and erectile dysfunction is a very important problem causing severe consequences in the individual suffering from it. Especially in diabetic patients this disorders are quite common and especially here the usual treatment, sildenafil, is not sufficient, therefore there is a clear medical need.

Therefore, the invention is a pharmaceutical composition comprising a compound A selected from PDE5 Inhibitors and at least one compound B selected from large conductance Ca(2+) activated K+ channels activator.

This composition was surprisingly showing extremely good results especially compared to e.g. sildenafil alone in diabetic models.

Ther term "PDE5-Inhibitor" is defining compounds inhibiting the function of Phosphodiesterase type 5 significantly.

In a preferred aspect of the invention the PDE-5 Inhibitor is selected from sildenafil, vardenafil, tadalafil or dipyridamole or any of their physiologically acceptable salts, hydrates or solvates
In a preferred aspect of the invention the PDE-5 Inhibitor is selected from sildenafil or any of its physiologically acceptable salts, hydrates or solvates

In a preferred aspect of the according to the invention the pharmaceutical composition is thus that the compound A is selected from sildenafil, sildenafil hydrochloride, sildenafil nitrate, sildenafil acetate, sildenafil hydrobromide, sildenafil lactate, sildenafil mesilate, sildenafil maleate, sildenafil fumarate, sildenafilsuccinate, sildenafil tatrate, sildenafil ascorbate and/or sildenafil citrate, most preferably sildenafil and sildenafil citrate.

In a preferred aspect of the according to the invention the pharmaceutical composition is thus that the compound B is selected from NS-1619, NS-8, NS-1608, NS-0004 and/or BMS-204352 or any of their physiologically acceptable salts, hydrates and solvates, most preferably NS-8 and NS-1619.

Another aspect of the invention is a Medicament comprising the pharmaceutical composition according to the invention as well as optionally at least one auxiliary material and/or additive and/or optionally another active ingredient.

Another aspect of the invention is the use of the pharmaceutical composition according to the invention, for the manufacture of a medicament for the treatment of sexual dysfunction in man and woman, sexual disorders in man, erectile dysfunction or impotence.

Sildenafil (1-[[3-(4,7-Dihydro-1-methyl-7-oxo-3-propyl-1*H*-pyrazolo[4,3-*d*]pyrimidin-5-yl)-4-ethoxyphenyl]sulfonyl]-4-methylpiperazine) is a well known compound first described in US 5,250,534 and EP 463,756, as well as EP_916675_B1 included here by reference.
Vardeanfil (2-[2-Ethoxy-5-(4-ethyl-piperazine-1-sulfonyl)-phenyl]-5-methyl-7-propyl-3H-imidazo[5,1-f][1,2,4]triazin-4-one) is also a well known PDE5 Inhibitor.

Tadalafil (6-Benzo[1,3]dioxol-5-yl-2-methyl-2,3,6,7,12,12a-hexahydro-pyrazino[1',2':1,6]pyrido[3,4-b]indole-1,4-dione) is also a well known PDE5 Inhibitor. Dipyridamole (2-[{6-[Bis-(2-hydroxy-ethyl)-amino]-4,8-di-piperidin-1-yl-pyrimido[5,4-d]pyrimid in-2-yl}-(2-hydroxy-ethyl)-amino]-ethanol) is also a well known PDE5 Inhibitor.

The term "salt" is to be understood as meaning any form of the active compound according to the invention in which this assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

The term "conductance Ca(2+) activated K+ channels activator" is also known as bk-channel openers and includes among others the compounds NS-1619, NS-8, NS-1608, NS-0004 and/or BMS-204352, their salts, hydrates and solvates. 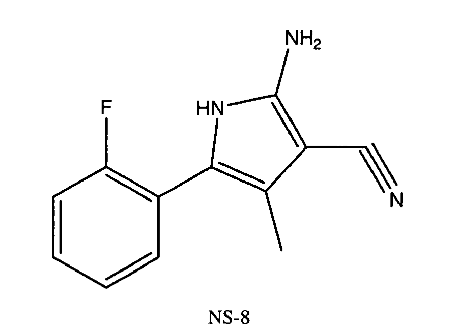 NS-8 (2-amino-5-(2-fluorophenyl)-4-methyl-1 H-pyrrole-3-carbonitrile or 2-amino-3-cyano-5-(2-fluorphenyl)-4-methylpyrrole) is a compound first described by NIPPON Shinyaku. Information about the compound including synthesis and formulation can be found in WO 99/61016, AU9939538, EP1283040-A1, EP1369416-A1 WO96/40634, EP842923, WO99/36068 or EP1057485 A1 all included here by reference. 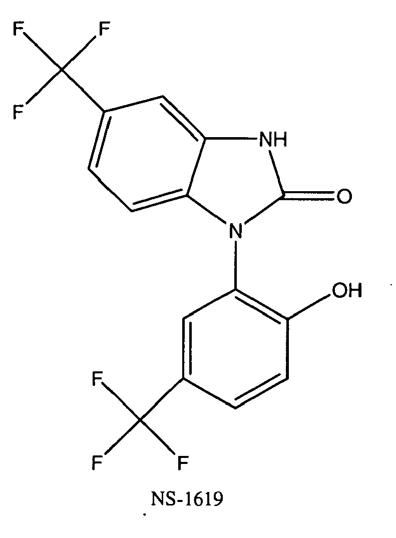 NS-1619 (5-(trifluoromethyl)-1-(5-(trifluoromethyl)-2-hydroxyphenyl)-1H-benzo[d]imidazol-2(3H)-one or 1,3-dihydro-1- [2-hydroxy5 (trifluoromethyl) phenyl] 5-(trifluoromethyl) 2-Hbenimidazol-one). Information about the compound is well known in the art. 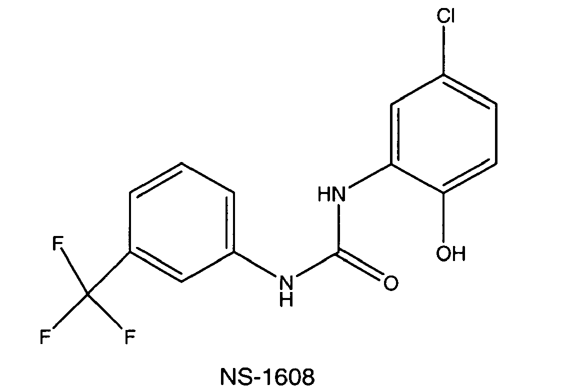 NS-1608 (1-(5-chloro-2-hydroxyphenyl)-3-(3-(trifluoromethyl)phenyl)urea) together with its analogue NS-0004. Information about the compound is well known in the art.

BMS-204352 (3-(5-Chloro-2-methoxy-phenyl)-3-fluoro-6-trifluoromethyl-1,3-dihydro-indol-2-one) also known as flindokalner is currently under development for stroke. Information about the compound is well known in the art.

In another aspect of the invention the "conductance Ca(2+) activated K+ channels activator" maybe, were applicable also be selected from this general list of potassium channel openers or agonists, which are selected from nicorandil, diazoxide, minoxidil, pinicadil, aprikalim, cromokulim, amlodipine, Bay K 8644 (L-type) (1,4-dihydro-26-dimethyl-5-nitro- 4 [2 (trifluoromethyl) phenyl]-3-pyridine carboxylic acid (methyl ester)), bepridil HCI (L-type), calciseptine (L-type), omega-conotoxin GVIA (N-type), omegaconotoxin MVIIC (Q-type), cyproheptadine HCI, dantrolene sodium (Ca2+ release inhibitor), diltiazem HCl (L-type), filodipine, flunarizine HCI (Ca2+/Na+), fluspirilene (L-type), HA-1077 2HCI (1- (5 isoquinolinyl sulphonyl) homo piperazine. HCl), isradipine, loperamide HCI, manoalide (Ca2 release inhibitor), nicardipine HCI (L-type), nifedipine (L-type), niguldipine HCl (Ltype), nimodipine (L-type), nitrendipine (L-type), pimozide (L-and T-type), ruthenium red, ryanodine (SR channels), taicatoxin, verapamil HCl (L-type), methoxy-verapamil HCl (L-type), YS-035 HCl (L-type) N [2 (3,4dimethoxyphenyl) ethyl]-3,4-dimethoxy N-methyl benzene ethaneamine HCl) and AV blockers such as verapamil and adenosine. It will be appreciated that this list includes calcium antagonists as potassium channel openers are indirect calcium antagonists.
In any case this list does not contain only "conductance Ca(2+) activated K+ channels activators" but also other substances which are considered not suitable.

Any medicament according to the invention contains the active ingredient as well as optionally at least one auxiliary material and/or additive and/or optionally another active ingredient.

The auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

Routes of administration can include intramuscular injection, intraveneous injection, subcutaneous injection, sublingual, bucal, patch through skin, oral ingestion, implantable osmotic pump, collagen implants, aerosols or suppository.

Included in this invention are especially also methods of treatments of a patient or a mammal, including men, suffering from sexual dysfunction in man and woman, sexual disorders in man, erectile dysfunction or impotence using the pharmaceutical composition according to the invention.

Especially the combination of this invention is of interest for severe dysfunction like e.g. the circumstances caused by diabetes mellitus.

Another aspect of the invention includes the combination according to the invention in which the PDE-5 Inhibitor is used in a sub therapeutic dose. Surprisingly the combination was highly active, even though the single compounds were only low active.

The examples and figures in the following section describing pharmacological trials are merely illustrative and the invention cannot be considered in any way as being restricted to these applications.

### Figures:

Examples and figures can be seen from the accompanying drawings

### Examples:

### Example 1: Von Frey-Model

The von Frey model is a model for neuropathic pain especially

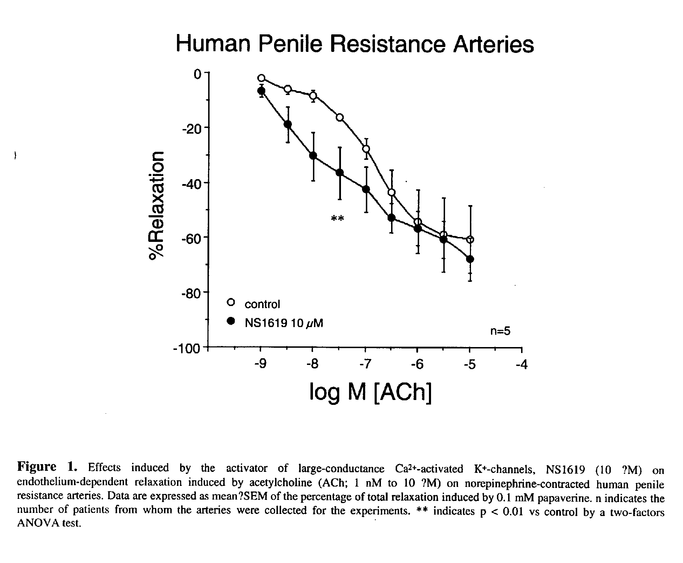

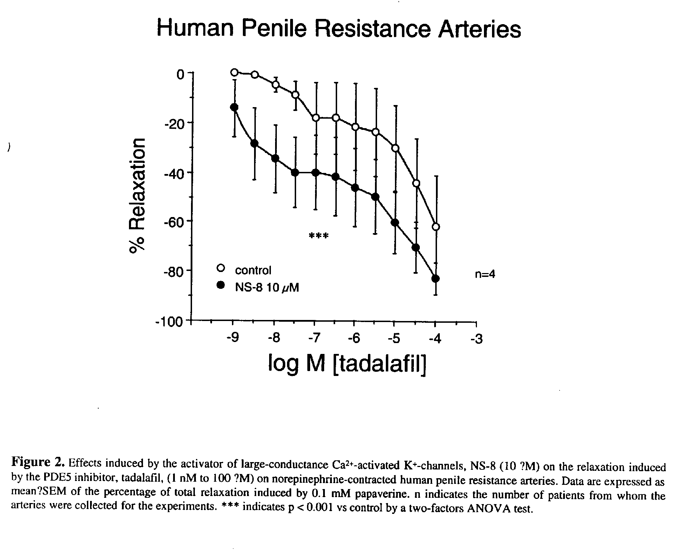

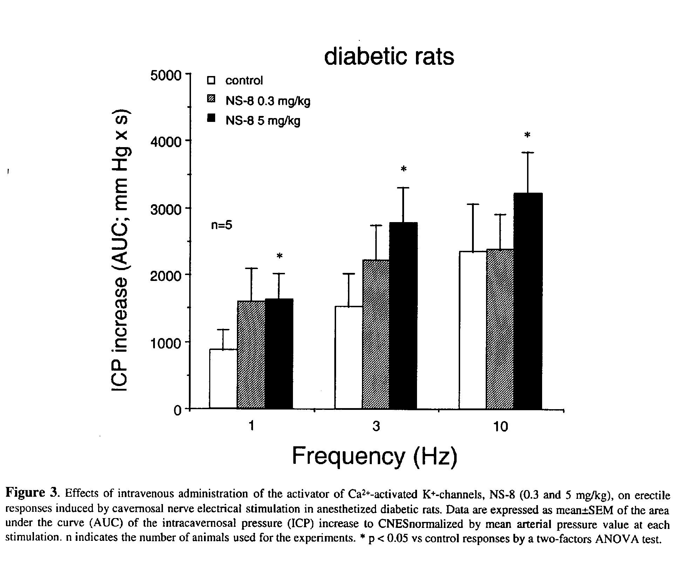

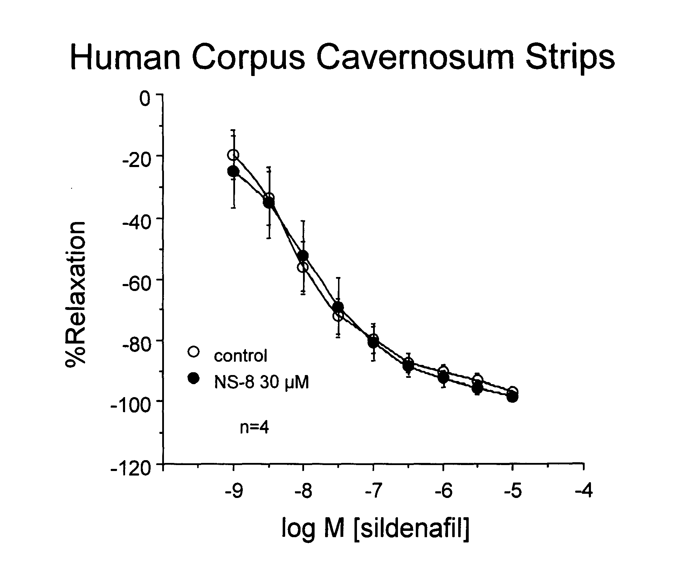

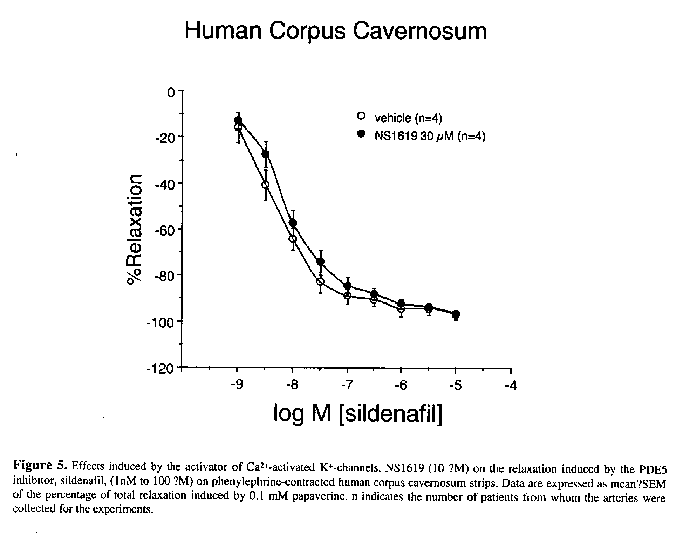

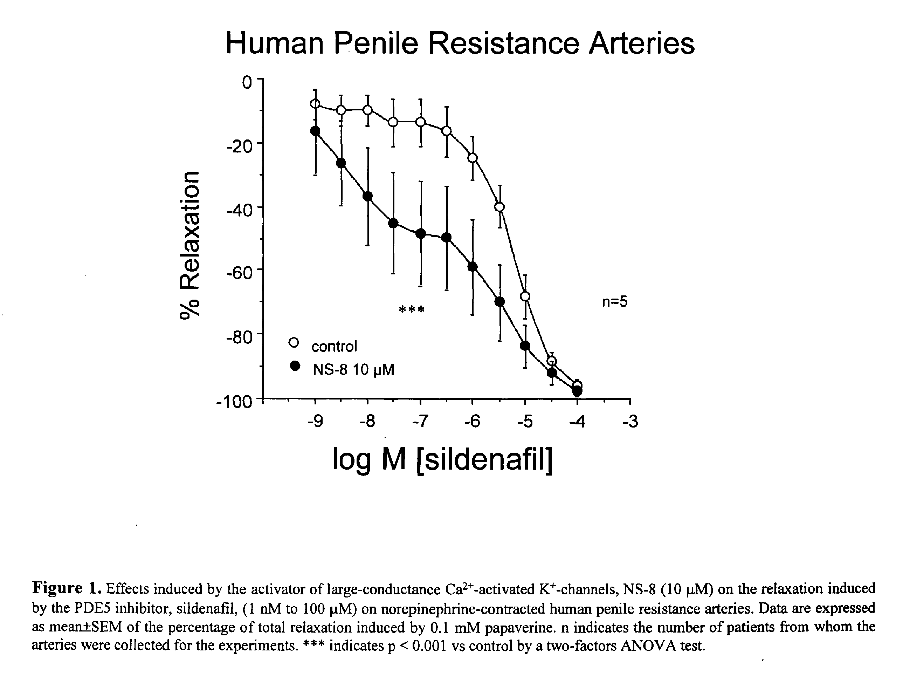

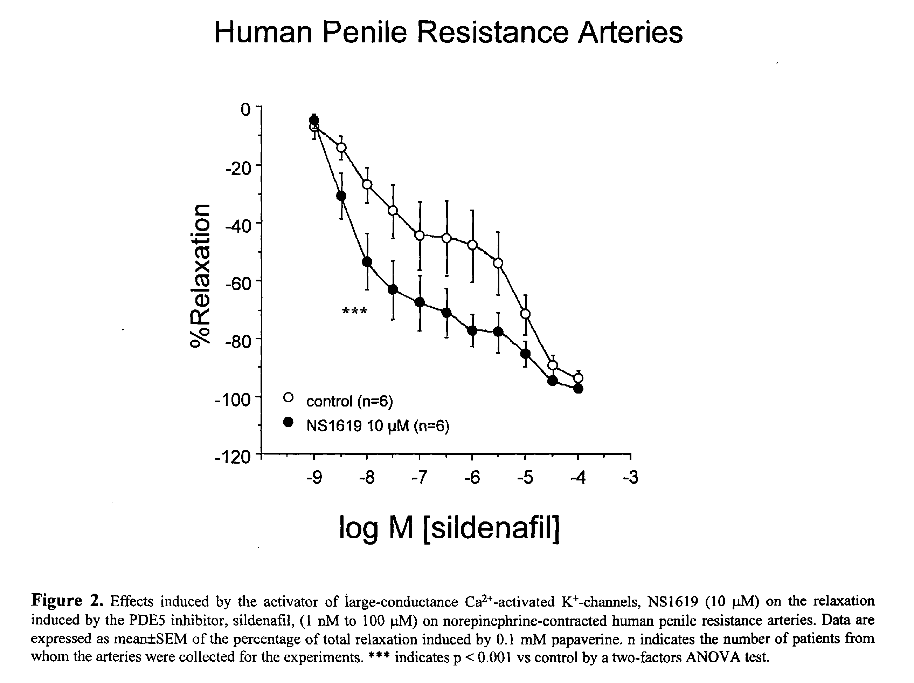

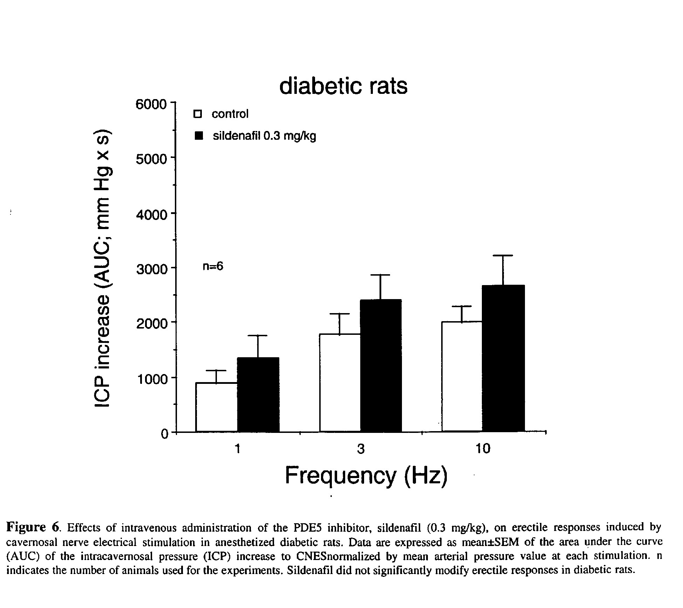

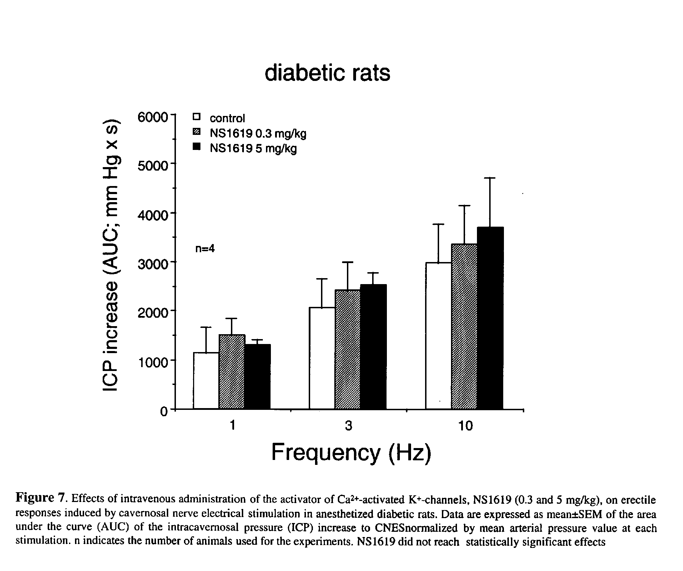

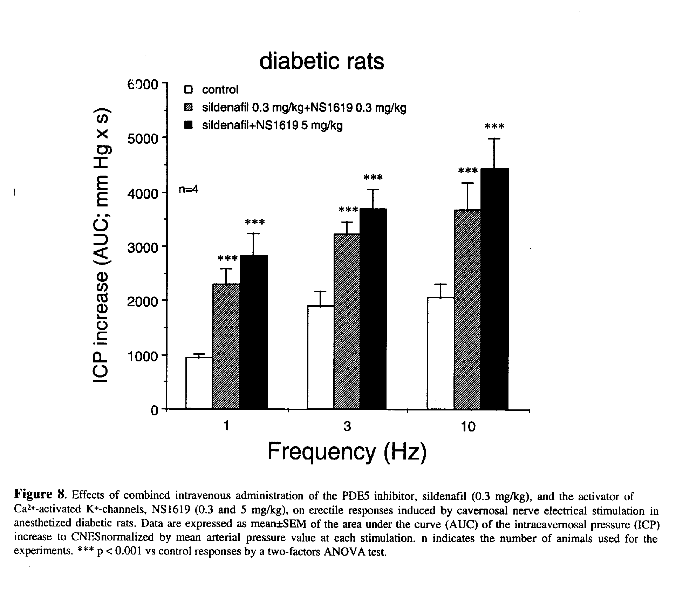

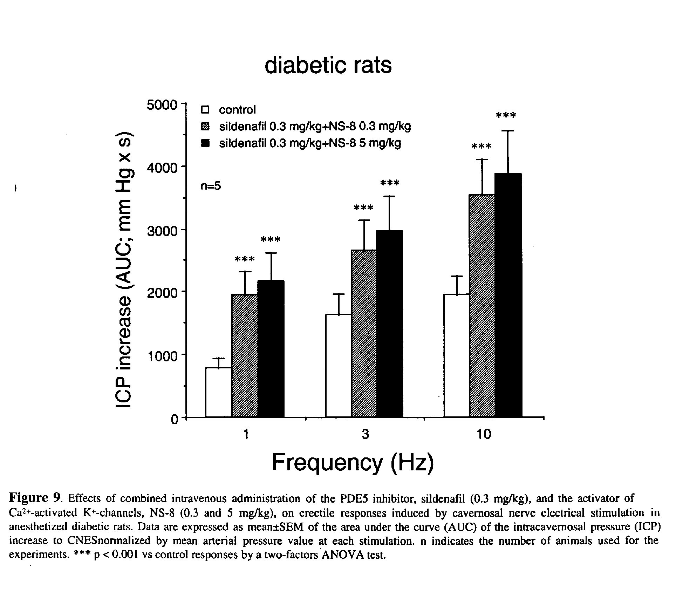

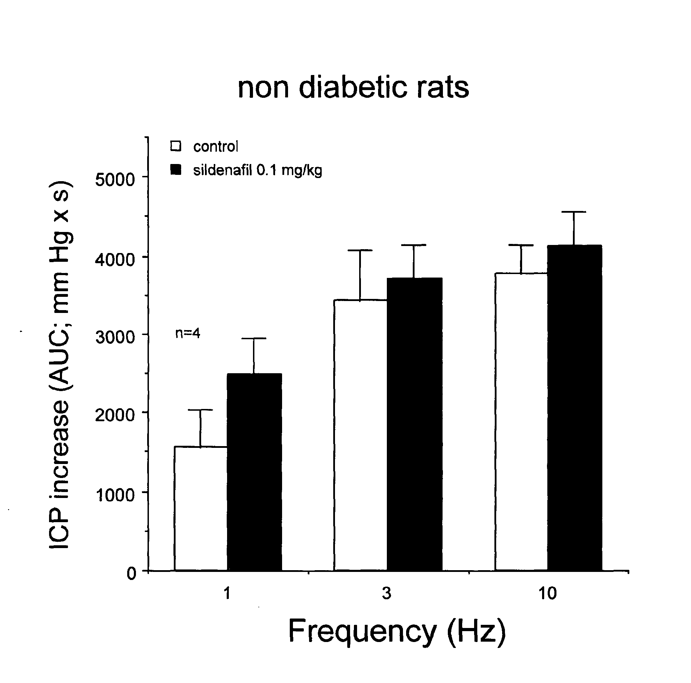

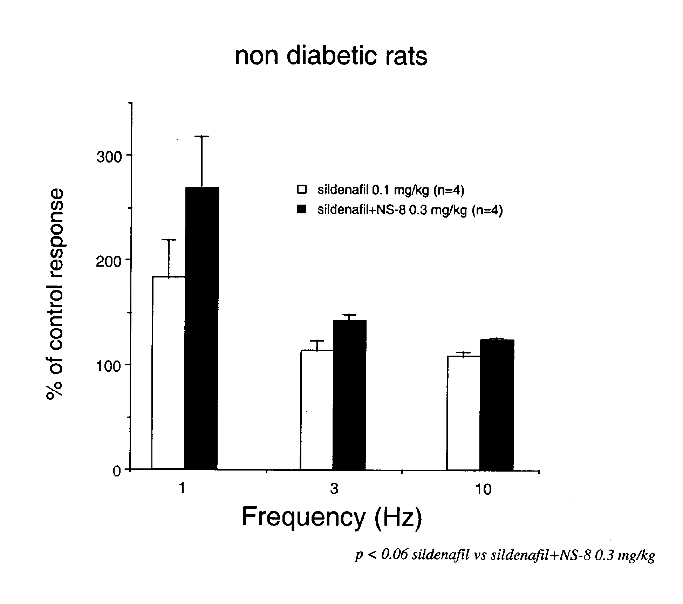

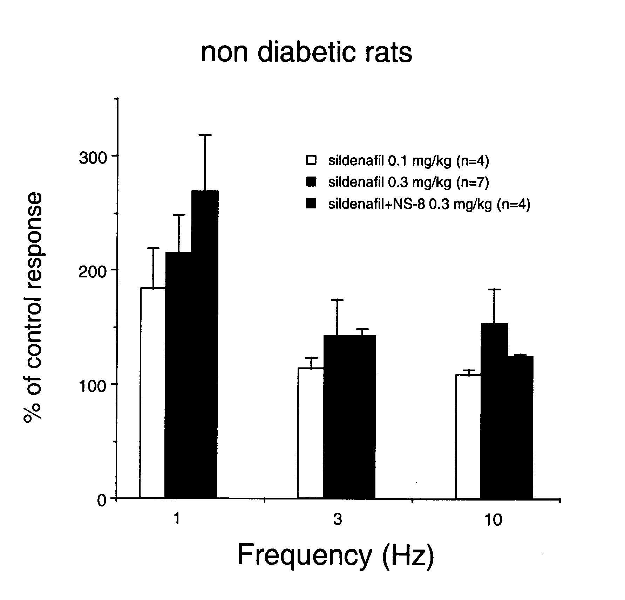

## Claims

1. A pharmaceutical composition comprising a compound A selected from a PDE5-Inhibitor and at least one compound B selected from large conductance Ca(2+) activated K+ channels activator.

2. A pharmaceutical composition according to claim 1 wherein the compound A is selected from sildenafil, vardenafil, tadalafil and dipyriamole or any of their physiologically acceptable salts, hydrates or solvates.

3. A pharmaceutical composition according to claim 2 wherein the compound A is selected from sildenafil or any of its physiologically acceptable salts, hydrates or solvates.

4. A pharmaceutical composition according to claim 3 wherein the compound A is selected from sildenafil, sildenafil hydrochloride, sildenafil nitrate, sildenafil acetate, sildenafil hydrobromide, sildenafil lactate, sildenafil mesilate, sildenafil maleate, sildenafil fumarate, sildenafilsuccinate, sildenafil tatrate, sildenafil ascorbate and/or sildenafil citrate, most preferably sildenafil and sildenafil citrate.

5. A pharmaceutical composition according to any of claims 1 to 4 wherein the compound A is included in a sub therapeutic concentration or amount.

6. A pharmaceutical composition according to any of claims 1 to 5 wherein the compound B is selected from NS-1619, NS-8, NS-1608, NS-0004 and/or BMS-204352 or any of their physiologically acceptable salts, hydrates and solvates, most preferably NS-8 and NS-1619.

7. Medicament comprising the pharmaceutical composition according to any of claims 1 to 6 as well as optionally at least one auxiliary material and/or additive and/or optionally another active ingredient.

8. Use of the pharmaceutical composition according to any of claims 1 to 6 for the manufacture of a medicament for the treatment of sexual dysfunction in man and woman, sexual disorders in man, erectile dysfunction or impotence.
